# EUROPÄISCHE PATENTSCHRIFT

(11) **EP 4 424 286 B1**
(45) Veröffentlichungstag und Bekanntmachung des Hinweises auf die Patenterteilung: **16.07.2025**
(21) Anmeldenummer: 24190507.4
(22) Anmeldetag: 28.09.2015
(51) Int. Cl.: A61F 9/008

(54) **PLANUNGSEINRICHTUNG UND VERFAHREN ZUM ERZEUGEN VON STEUERDATEN FÜR EINE AUGENCHIRURGISCHE BEHANDLUNGSVORRICHTUNG**
PLANNING DEVICE AND METHOD FOR GENERATING CONTROL DATA FOR AN EYE SURGICAL TREATMENT DEVICE
DISPOSITIF DE PLANIFICATION ET PROCÉDÉ DE GÉNÉRATION DE DONNÉES DE COMMANDE POUR UN DISPOSITIF DE TRAITEMENT CHIRURGICAL OPHTALMIQUE

(30) Priorität: 29.09.2014 DE 102014014566
(43) Veröffentlichungstag der Anmeldung: 04.09.2024
(62) Teilanmeldung aus: 15770549.2
(73) Patentinhaber: Carl Zeiss Meditec AG, 07745 Jena (DE)
(72) Erfinder: Bischoff, Mark, 07745 Jena (DE); Stobrawa, Gregor, 07745 Jena (DE)
(74) Vertreter: Rößner, Ulrike

(56) Entgegenhaltungen:
- DE-A1- 102007 019 815
- DE-A1- 102013 218 415
- US-A1- 2009 157 061
- US-A1- 2012 172 854
- US-A1- 2014 264 980

## Beschreibung

Die Erfindung bezieht sich auf eine Planungseinrichtung zum Erzeugen von Steuerdaten für eine Behandlungsvorrichtung, die mittels einer Lasereinrichtung zumindest einen Schnitt in der Hornhaut erzeugt. Die Erfindung bezieht sich weiter auf eine Behandlungsvorrichtung, die eine Planungseinrichtung der genannten Art aufweist.

Die Erfindung bezieht sich weiter auf ein Verfahren zum Erzeugen von Steuerdaten für eine Behandlungsvorrichtung, die mittels einer Lasereinrichtung zumindest einen Schnitt in der Hornhaut erzeugt.

Beschrieben wird auch ein Verfahren zur Augenchirurgie, das nicht Teil der Erfindung ist, wobei mittels einer Behandlungsvorrichtung mit einer Lasereinrichtung zumindest einen Schnitt in der Hornhaut erzeugt wird.

Im Stand der Technik sind verschiedenste Behandlungsverfahren mit dem Ziel der Refraktionskorrektur am menschlichen Auge bekannt. Ziel der Operationsmethoden ist es dabei, die Hornhaut gezielt zu verändern, um so die Lichtbrechung im Auge zu beeinflussen. Hierfür werden mehrere Operationsmethoden eingesetzt. Am verbreitetsten ist gegenwärtig die sogenannte Laser-Insitu-Keratomileusis, die auch LASIK abgekürzt wird. Dabei wird zuerst eine Hornhaut-Lamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp., Irvine, USA, vertrieben wird. Nachdem die Lamelle gelöst und zur Seite geklappt wurde, ist bei der LASIK- Operation die Anwendung eines Excimer-Lasers vorgesehen, der das derart unter der Lamelle freigelegte Hornhautgewebe durch Ablation abträgt. Nachdem auf diese Art und Weise unter der Hornhautoberfläche liegendes Volumen verdampft wurde, wird die Hornhaut-Lamelle wieder auf den ursprünglichen Platz zurückgeklappt.

Die Anwendung eines Laserkeratoms zum Freilegen der Lamelle ist gegenüber einem mechanischen Messer vorteilhaft, da die geometrische Präzision verbessert und die Häufigkeit klinisch relevanter Komplikationen verringert ist. Insbesondere kann die Lamelle mit sehr viel konstanterer Dicke hergestellt werden, wenn Laserstrahlung verwendet wird. Auch ist die Schnittkannte präzise geformt, was die Gefahr für Heilungsstörungen durch diese auch nach der Operation verbleibende Grenzfläche mindert. Nachteilig bei diesem Verfahren ist allerdings, dass zwei unterschiedliche Behandlungsvorrichtungen verwendet werden müssen, zum einen nämlich das Laserkeratom zum Freilegen der Lamelle und zum anderen der das Hornhautgewebe verdampfende Laser.

Diese Nachteile sind behoben bei einem Verfahren, das jüngst durch die Carl Zeiss Meditec AG implementiert wurde. Bei diesem Verfahren, welches allgemein als Lentikelextraktion bezeichnet wird, wird mittels eines Kurzpulslasers, vorzugsweise eines Femtosekundenlasers in der Augenhornhaut eine Schnittgeometrie gebildet, welche in der Hornhaut ein Hornhaut-Volumen (sog. Lentikel) separiert. Dieses wird dann manuell vom Operateur entnommen. Der Vorteil dieses Verfahrens liegt zum einen darin, dass die Schnittqualität durch Anwendung des Femtosekundenlasers nochmals verbessert ist.

Zum anderen ist nur noch eine Behandlungsvorrichtung erforderlich; der Excimer-Laser wird nicht mehr eingesetzt.

Eine medizinisch vorteilhafte Variante des Verfahrens wird in der Literatur als SMILE-Verfahren bezeichnet, bei dem kein Flap erzeugt wird, sondern nur ein kleiner Öffnungsschnitt als Zugang zu dem unter dem sogenannten Cap liegenden Lentikel dient. Das separierte Lentikel wird durch diesen kleinen Öffnungsschnitt entnommen, wodurch die biomechanische Integrität der vorderen Hornhaut weniger beeinträchtigt wird als bei anderen Verfahren. Hinzu kommt, dass auf diese Weise weniger oberflächliche Nervenfasern in der Hornhaut zerschnitten werden, was sich nachweislich günstig auf die Wiederherstellung der ursprünglichen Sensibilität der Hornhautoberfläche auswirkt. Das nach LASIK oft zu behandelnde Symptom trockener Augen ist dadurch in seiner Ausprägung und Dauer reduziert. Auch andere Komplikationen nach LASIK, die meist mit dem Flap im Zusammenhang stehen (z.B. Falten, Epithel-Einwachsungen im Flapbett) treten ohne Flap seltener auf.

Bei der Erzeugung von Schnitten in der Hornhaut mittels Laserstrahlung wird üblicherweise die optische Strahlungswirkung dadurch ausgenutzt, dass ein optischer Durchbruch durch einzelne optische Pulse, deren Dauer zwischen etwa 100 fs und 100 ns liegen kann, erzeugt wird. Auch ist es bekannt, einzelne Pulse, deren Energie unter einem Schwellwert für einen optischen Durchbruch liegt, derart überdeckt ins Gewebe bzw. Material einzubringen, dass auch damit eine Material- bzw. Gewebetrennung erreicht wird. Dieses Konzept der Schnitterzeugung im Hornhautgewebe erlaubt eine große Vielfalt an Schnitten.

Es ist ebenfalls Stand der Technik, eine Refraktionskorrektur durch die Einbringung eines Implantats (auch Inlay genannt) in die Hornhaut zumindest eines Auges eines betroffenen Patienten vorzunehmen. Dabei kann das Implantat künstlicher Natur sein, z.B. ein Ring oder eine Linse aus einem Kunststoffmaterial (z.B. KAMRA^{®}, Flexivue^{®}), oder auch ein entsprechend geformtes Implantat aus einem Biomaterial oder ein Transplantat aus humanem Hornhautgewebe.

Es ist für die gegenwärtig verfügbaren Implantate hierzu üblich, mittels Femtosekunden-Laserkeratom eine taschenartige Schnittgeometrie (Pocket) in der Hornhaut zu erzeugen, welche zur Aufnahme des Implantats bestimmt ist und gleichzeitig die Einführung des Implantats durch den Arzt unterstützt.

Lentikelextraktionsverfahren bieten grundsätzlich gute Möglichkeiten, mit einem Implantationsverfahren kombiniert zu werden, indem im Anschluss an die Lentikelentnahme ein Implantat in das Innere der Hornhaut platziert wird. Das SMILE-Verfahren bietet zudem die Möglichkeit, die infolge des Verfahrens geschaffene taschenartige Schnittgeometrie für Aufnahme des Implantats zu nutzen und so ein gewisses Maß an mechanischer Stabilität zu bieten.

Es hat sich aber herausgestellt, dass der Erfolg einer solchen Refraktionskorrektur nicht immer den Erwartungen entspricht und nach der Behandlung ein (wenn auch geringerer) Refraktionsfehler verbleibt. Es ist hier gegenwärtig bereits machbar, bekannte chirurgische Korrekturverfahren (PRK oder LASIK mit Excimer-Laser) anzuwenden, um betroffene Augen vor oder nach der Implantation eines Inlays nochmals refraktiv zu korrigieren (Nachkorrektur).

Erfolgt die Korrektur mit einem der genannten Korrekturverfahren, so stellt der Abtrag von Gewebe, welches das Implantat überdeckt und auch die direkte Wechselwirkung der Therapiestrahlung mit dem Inlay ein gewisses Risiko für die Wirksamkeit und Sicherheit des Gesamtverfahrens dar.

Auf einem anderen Gebiet der Augenheilkunde, nämlich der Behandlung von Altersweitsichtigkeit (Presbyopie) ist es bekannt, in die Hornhaut konzentrische Schnitte einzubringen, um deren mechanische Stabilität zu verändern. Eine solche Lösung ist zum Beispiel in der US 7 717 907 beschrieben, wobei dort die Schnitte bis in eine Tiefe von 90% der Hornhautdicke gehen. Diese tiefen Schnitte bewirken Hornhaut-Krümmungsänderungen durch das Zusammenwirken von Augeninnendruck und reduzierten Gegenkräften in der gesamten Hornhaut (induzierter Keratokonus).

Der Erfindung liegt deshalb die Aufgabe zugrunde, eine Planungseinrichtung zum Erzeugen von Steuerdaten, eine Behandlungsvorrichtung zur Refraktion korrigierenden Augenchirurgie sowie ein Verfahren zum Erzeugen von Steuerdaten für eine solche Behandlungsvorrichtung anzugeben, bei dem eine sichere Nachkorrektur nach Versorgung mit einem Hornhautimplantat gewährleistet ist.

Diese Aufgabe wird erfindungsgemäß mit einer Planungseinrichtung der eingangs genannten Art gelöst, die Berechnungsmittel zum Festlegen mindestens eines Hornhaut-Schnitts aufweist, wobei die Berechnungsmittel die Hornhaut-Schnitte so bestimmen, dass den nach Einbringen eines Implantats in die Hornhaut bestehenden Refraktionsfehlern entgegengewirkt wird.

Die Erfindung wird weiter gelöst mit einer Behandlungsvorrichtung, die eine Lasereinrichtung aufweist, welche mittels Laserstrahlung gemäß Steuerdaten zumindest einen Schnitt in der Hornhaut vornimmt, und eine Planungseinrichtung nach der soeben genannten Art zum Erzeugen der Steuerdaten aufweist, wobei die Planungseinrichtung die Hornhaut-Schnitte so bestimmt, dass den nach Einbringen eines Implantats in die Hornhaut bestehenden Refraktionsfehlern entgegengewirkt wird.

Die Erfindung wird schließlich ebenfalls gelöst mit einem Verfahren zum Erzeugen von Steuerdaten gemäß der eingangs genannten Art, das aufweist: Erzeugen eines Steuerdatensatzes für die Hornhaut-Schnitte zur Ansteuerung der Lasereinrichtung, wobei die Planungseinrichtung die neuen Hornhaut-Schnitte so bestimmt, dass nach dem Einbringen eines Implantats in die Hornhaut bestehenden Refraktionsfehlern entgegengewirkt wird.

Weiter wird ein nichtbeanspruchtes Verfahren beschrieben, das umfasst: Erzeugen eines Steuerdatensatzes für die Hornhaut-Schnitte, Übertragen der Steuerdaten zur Behandlungsvorrichtung und Erzeugen der Schnitte durch Ansteuern der Lasereinrichtung mit dem Steuerdatensatz, wobei beim Erzeugen des Steuerdatensatzes die neue Hornhaut-Schnitt so bestimmt werden, dass den nach Einbringen eines Implantats in die Hornhaut bestehenden Refraktionsfehlern entgegengewirkt wird.

Eine dazu geeignete Vorrichtung ist in der DE 10 2007 019 815 A1 sowie in der DE 10 2013 218 415 A1 der Anmelderin beschrieben. In der US 2012/172854 A1 sind Hornhaut-Entlastungschnitte im Zusammenhang mit Hornhauttransplantationen erwähnt.

Das erfindungsgemäße Verfahren und die entsprechende Vorrichtung bewirkt Änderungen der Form der Hornhaut durch das Zusammenwirken der vom implantierten Lentikel (Implantat) hervorgerufenen Kräfte und der reduzierten Gegenkräfte in der vorderen Hornhaut des Caps, d.h. dem über dem Implantat liegenden Hornhautteils.

Diese Schnitte (im Folgenden auch Entlastungsschnitte genannt) werden insbesondere in der Nähe der Bowman-Membran eingebracht und können diese durchschneiden. Sie reichen nicht tiefer als bis zum Pocket-Schnitt, also nicht tiefer als bis in die Tiefe des Implantats.

Dabei ist es vorteilhaft, wenn die Schnitte nicht tiefer als 80% der Dicke des Caps in die Hornhaut hineinreichen. Weiter ist es günstig, wenn die Schnitte vollständig im Inneren der Hornhaut verlaufen. Auf diese Weise wird vermieden, dass im darüber liegenden Gewebe Öffnungen entstehen, die sich ungünstig auf den Heilungsverlauf auswirken können.

Die Entlastungsschnitte werden also möglichst so gestaltet, dass bei eventuellem Aufklaffen Epithelzellen nicht tief einwandern können. Dies kann auch dadurch verbessert werden, dass die Schnitte nicht oder nicht überall senkrecht zur Oberfläche ausgerichtet sind. Dies kann ebenfalls dadurch verbessert werden, dass die Schnitte nicht oder nicht überall radial oder zirkulär bezüglich der Hornhautmitte ausgerichtet sind.

Die Erfindung ist nicht nur für die Verbesserung der Hyperopiekorrektur geeignet. In analoger Weise ist sie auch für eine Myopiekorrektur mittels Lentikeltransplantation anwendbar.

Die Erfindung kann in Verbindung mit Implantaten aus Kunststoff oder Biomaterial aber auch in Verbindung mit Transplantaten, insbesondere mit transplantierten Lentikeln angewendet werden.

Die Erfindung ist nicht auf die im weiteren dargestellten Schnittformen beschränkt. So lange die Schnitte hinsichtlich Zweckbestimmung, Wirkmechanismus und ungefährer Lage übereinstimmen, wird das Verfahren ihrer Planung, Erzeugung, Implementierung und die entsprechenden Vorrichtungen ebenfalls beansprucht. Weitere Schnitte können z. B. auch zur Astigmatismuskorrektur oder Korrektur von Sehfehlern höherer Ordnung eingebracht werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
Fig. 1 eine schematische Darstellung einer Behandlungsvorrichtung mit einer Planungseinrichtung für eine Nachbehandlung bei augenchirurgischer Refraktionskorrektur,
Fig. 2 eine schematische Darstellung der Wirkung der Laserstrahlung, die in der Behandlungsvorrichtung der Fig. 1 verwendet wird,
Fig. 3 eine weitere Schemadarstellung des Behandlungsgerätes der Fig. 1 hinsichtlich der Einbringung der Laserstrahlung,
Fig. 4 eine schematische Schnittdarstellung durch die Augenhornhaut zur Veranschaulichung der Entnahme eines Hornhaut-Volumens im Zusammenhang mit einer augenchirurgischen Refraktionskorrektur nach dem Stand der Technik,
Fig. 5 eine Schemadarstellung hinsichtlich des Aufbaus des Behandlungsgerätes der Fig. 1 mit besonderem Bezug auf die dort vorhandene Planungseinrichtung,
Fig. 6 eine Schemadarstellung zur Veranschaulichung der Einbringung eines Inlays im Zusammenhang mit einer augenchirurgischen Refraktionskorrektur nach dem Stand der Technik
Fig. 7 eine Schemadarstellung zur Veranschaulichung der Erfindung
Fig. 8 eine Schemadarstellung zur Veranschaulichung der Erfindung in einer zweiten Ausführungsform
Fig. 9 eine Schemadarstellung zur Veranschaulichung der Erfindung in einer weiteren Ausführungsform
Fig. 10 eine Schemadarstellung zur Veranschaulichung der Erfindung in einer weiteren Ausführungsform in Zusammenhang mit einer Astigmatismuskorrektur
Fig. 11 eine Schemadarstellung verschiedener weiterer Schnittgeometrien.

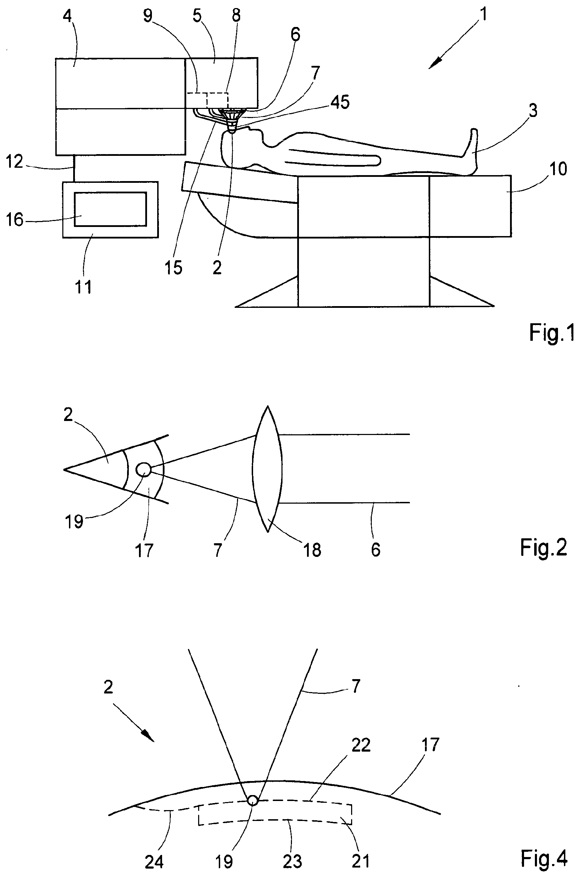

Eine Behandlungsvorrichtung für die Augenchirurgie ist in Fig. 1 dargestellt und mit dem allgemeinen Bezugszeichen 1 versehen. Die Behandlungsvorrichtung 1 ist für die Einbringung von Laserschnitten an einem Auge 2 eines Patienten 3 ausgebildet. Dazu weist die Behandlungsvorrichtung 1 eine Lasereinrichtung 4 auf, die aus einer Laserquelle 5 einen Laserstrahl 6 abgibt, welcher als fokussierter Strahl 7 in das Auge 2 bzw. die Augenhornhaut gerichtet wird. Vorzugsweise ist der Laserstrahl 6 ein gepulster Laserstrahl mit einer Wellenlänge zwischen 300 Nanometer und 10 Mikrometer. Weiter liegt die Pulslänge des Laserstrahls 6 im Bereich zwischen 1 Femtosekunde und 100 Nanosekunden, wobei Pulswiederholraten von 50 bis 50000 Kilohertz und Pulsenergien zwischen 0,01 Mikrojoule und 0,01 Millijoule möglich sind. Die Behandlungsvorrichtung 1 erzeugt somit in der Hornhaut des Auges 2 durch Ablenkung der gepulsten Laserstrahlung eine Schnittfläche. In der Lasereinrichtung 4 bzw. deren Laserquelle 5 ist deshalb dazu noch ein Scanner 8 sowie ein Strahlungsintensitätsmodulator 9 vorgesehen.

Der Patient 3 befindet sich auf einer Liege 10, die in drei Raumrichtungen verstellbar ist, um das Auge 2 passend zum Einfall des Laserstrahls 6 auszurichten. In bevorzugter Bauweise ist die Liege 10 motorisch verstellbar.

Die Ansteuerung kann insbesondere durch ein Steuergerät 11 erfolgen, das grundsätzlich den Betrieb der Behandlungsvorrichtung 1 steuert und dazu über geeignete Datenverbindungen, beispielsweise Verbindungsleitungen 12 mit der Behandlungsvorrichtung verbunden ist. Natürlich kann diese Kommunikation auch über andere Wege, beispielsweise Lichtleiter oder per Funk geschehen. Das Steuergerät 11 nimmt die entsprechenden Einstellungen, Zeitsteuerung an der Behandlungsvorrichtung 1, insbesondere der Lasereinrichtung 4 vor und bewerkstelligt damit entsprechende Funktionen der Behandlungsvorrichtung 1.

Die Behandlungsvorrichtung 1 weist weiter noch eine Fixiereinrichtung 15 auf, welche die Hornhaut des Auges 2 gegenüber der Lasereinrichtung 4 lagefixiert. Diese Fixiereinrichtung 15 kann dabei ein bekanntes Kontaktglas 45 umfassen, an das die Augenhornhaut durch Unterdruck angelegt wird und das der Augenhornhaut eine gewünschte geometrische Form verleiht. Solche Kontaktgläser sind dem Fachmann aus dem Stand der Technik bekannt, beispielsweise aus der DE 102005040338 A1.

Die Behandlungseinrichtung 1 weist weiterhin eine hier nicht dargestellte Kamera auf, welche durch das Kontaktglas 45 hindurch ein Bild der Augenhornhaut 17 aufnehmen kann. Dabei kann die Beleuchtung für die Kamera sowohl im sichtbaren als auch im infraroten Bereich des Lichtes erfolgen.

Das Steuergerät 11 der Behandlungsvorrichtung 1 weist weiter noch eine Planungseinrichtung 16 auf, die später noch näher erläutert werden wird.

Fig. 2 zeigt schematisch die Wirkungsweise des einfallenden Laserstrahls 6. Der Laserstrahl 6 wird fokussiert und fällt als der fokussierte Laserstrahl 7 in die Hornhaut 17 des Auges 2. Zur Fokussierung ist eine schematisch eingezeichnete Optik 18 vorgesehen. Sie bewirkt in der Hornhaut 17 einen Fokus, in dem die Laserstrahlungsenergiedichte so hoch ist, dass in Kombination mit der Pulslänge der gepulsten Laserstrahlung 6 ein nicht-linearer Effekt in der Hornhaut 17 auftritt. Beispielsweise kann jeder Puls der gepulsten Laserstrahlung 6 im Fokus 19 einen optischen Durchbruch in der Augenhornhaut 17 erzeugen, welche wiederum eine in Fig. 2 nur schematisch angedeutete Plasmablase initiiert. Bei Entstehung der Plasmablase umfasst die Gewebsschichttrennung ein größeres Gebiet als den Fokus 19, obwohl die Bedingungen zur Erzeugung des optischen Durchbruches nur im Fokus 19 erreicht werden. Damit von jedem Laserpuls ein optischer Durchbruch erzeugt wird, muss die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt. Alternativ kann ein gewebetrennender Effekt auch durch gepulste Laserstrahlung erreicht werden, indem mehrere Laserstrahlungspulse in einem Bereich abgegeben werden, wobei sich die Fokus-Spots überlappen. Es wirken dann mehrere Laserstrahlungspulse zusammen, um einen gewebetrennenden Effekt zu erreichen. Die Art der Gewebetrennung, die die Behandlungsvorrichtung 1 einsetzt, ist jedoch für die nachfolgende Beschreibung nicht weiter relevant; wesentlich ist lediglich, dass eine Schnitterzeugung in der Hornhaut 17 des Auges 2 stattfindet.

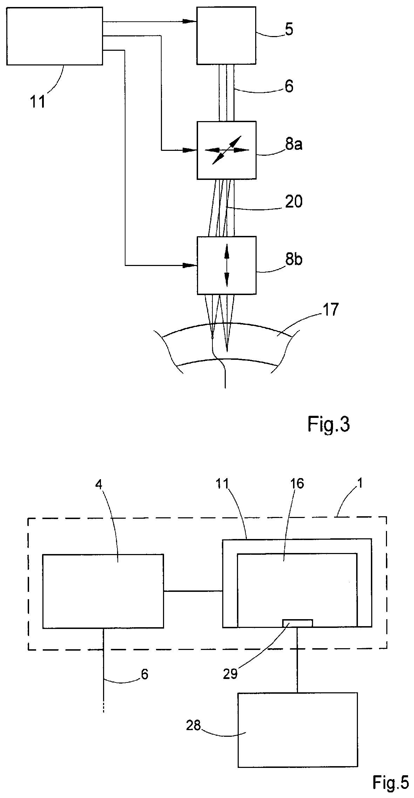

Um eine augenchirurgische Refraktionskorrektur auszuführen, wird mittels der Laserstrahlung 6 aus einem Gebiet innerhalb der Hornhaut 17 ein Hornhautvolumen entfernt, indem dort Gewebeschichten getrennt werden, die das Hornhaut-Volumen isolieren und dann dessen Entnahme ermöglichen. Zur Isolierung des zu entfernenden Hornhaut-Volumens wird z.B. im Falle der gepulst eingebrachten Laserstrahlung die Lage des Fokus 17 der fokussierten Laserstrahlung 7 in der Hornhaut 17 verstellt. Dies ist schematisch in Fig. 3 gezeigt. Die Brechungseigenschaften der Hornhaut 17 werden durch die Entnahme des Volumens gezielt verändert, um so die Refraktionskorrektur zu erreichen. Das Volumen ist deshalb meist linsenförmig und wird als Lentikel bezeichnet. Für die vorliegende Erfindung reicht es jedoch aus, dass die Gewebeschichten so getrennt werden, dass eine Tasche zur Aufnahme des Implantats entsteht.

In Fig. 3 sind die Elemente der Behandlungsvorrichtung 1 nur insoweit eingetragen, als sie zum Verständnis der Schnitterzeugung erforderlich sind. Der Laserstrahl 6 wird, wie bereits erwähnt, in einem Fokus 19 in der Hornhaut 19 gebündelt, und die Lage des Fokus 19 in der Hornhaut wird verstellt, so dass zur Schnitterzeugung an verschiedenen Stellen fokussierende Energie aus Laserstrahlungspulsen in das Gewebe der Hornhaut 17 eingetragen wird. Die Laserstrahlung 6 wird von der Laserquelle 5 vorzugsweise als gepulste Strahlung bereitgestellt. Der Scanner 8 ist in der Bauweise der Fig. 3 zweiteilig aufgebaut und besteht aus einem xy-Scanner 8a, der in einer Variante durch zwei im Wesentlichen orthogonal ablenkende Galvanometerspiegel realisiert ist. Der Scanner 8a lenkt den von der Laserquelle 5 kommenden Laserstrahl 6 zweidimensional ab, so dass nach dem Scanner 9 ein abgelenkter Laserstrahl 20 vorliegt. Der Scanner 8a bewirkt somit eine Verstellung der Lage des Fokus 19 im Wesentlichen senkrecht zur Haupteinfallsrichtung des Laserstrahls 6 in der Hornhaut 17. Zur Verstellung der Tiefenlage ist neben dem xy-Scanner 8a im Scanner 8 noch ein z-Scanner 8b vorgesehen, der beispielsweise als verstellbares Teleskop ausgebildet ist. Der z-Scanner 8b sorgt dafür, dass die z-Position der Lage des Fokus 19, d.h. dessen Position auf der optischen Achse des Einfalls verändert wird. Der z-Scanner 8b kann dem xy-Scanner 8a nach- oder vorgeordnet sein.

Für das Funktionsprinzip der Behandlungsvorrichtung 1 ist die Zuordnung der einzelnen Koordinaten zu dem Raumrichtungen nicht wesentlich, genau so wenig, dass der Scanner 8a um zueinander rechtwinklige Achsen ablenkt. Vielmehr kann jeder Scanner verwendet werden, der in der Lage ist, den Fokus 19 in einer Ebene zu verstellen, in der die Einfallsachse der optischen Strahlung nicht liegt. Weiter können auch beliebige nichtkartesische Koordinatensystem zur Ablenkung bzw. Steuerung der Lage des Fokus 19 verwendet werden. Beispiele dafür sind Kugelkoordinaten oder zylindrische Koordinaten. Die Steuerung der Lage des Fokus 19 erfolgt mittels der Scanner 8a, 8b unter Ansteuerung durch das Steuergerät 11, das entsprechende Einstellungen an der Laserquelle 5, dem (in Fig. 3 nicht gezeigten) Modulator 9 sowie dem Scanner 8 vornimmt. Das Steuergerät 11 sorgt für einen geeigneten Betrieb der Laserquelle 5 sowie die hier exemplarisch geschilderte dreidimensionale Fokusverstellung, so dass letztendlich eine Schnittfläche ausgebildet wird, die ein bestimmtes Hornhaut-Volumen isoliert, das zur Refraktionskorrektur entfernt werden soll.

Die Steuereinrichtung 11 arbeitet nach vorgegebenen Steuerdaten, welche beispielsweise bei der hier lediglich exemplarisch geschilderten Lasereinrichtung 4 als Zielpunkte für die Fokusverstellung vorgegeben sind. Die Steuerdaten sind in der Regel in einem Steuerdatensatz zusammengefasst. Dies ergibt geometrische Vorgaben für die auszubildende Schnittfläche, beispielsweise die Koordinaten der Zielpunkte als Muster vor. Der Steuerdatensatz enthält dann in dieser Ausführungsform auch konkrete Stellenwerte für den Fokuslagenverstellmechanismus, z.B. für den Scanner 8.

Die Erzeugung der Schnittfläche mit der Behandlungsvorrichtung 1 ist exemplarisch in Fig. 4 gezeigt. Ein Hornhaut-Volumen 21 in der Hornhaut 17 wird durch Verstellung des Fokus 19, in dem der fokussierte Strahl 7 gebündelt ist, isoliert. Dazu werden Schnittflächen ausgebildet, die hier exemplarisch als anteriore Flap-Schnittfläche 22 sowie als posteriore Lentikel-Schnittfläche 23 ausgebildet sind. Diese Begriffe sind hier lediglich exemplarisch zu verstehen und sollen den Bezug auf das herkömmliche Lasik- oder Lentikel-ExtraktionsVerfahren (SMILE) herstellen, für das die Behandlungsvorrichtung 1, wie bereits geschildert, ebenfalls ausgebildet ist. Wesentlich ist hier lediglich, dass die Schnittflächen 22 und 23 sowie nicht weiter bezeichnete Randschnitte, welche die Schnittflächen 22 und 23 an deren Rändern zusammenführen, das Hornhaut-Volumen 21 isolieren. Durch einen Öffnungsschnitt 24 kann weiter eine das Hornhaut-Volumen 21 anterior begrenzende Hornhaut-Lamelle abgeklappt werden, so dass das Hornhaut-Volumen 21 entnehmbar ist. Diese durch den anterioren Schnitt definierte Hornhaut-Lamelle hat in der bevorzugten Ausführung eine konstante Dicke, kann aber auch eine inhomogene Dicke habe, insbesondere eine radiusabhängige Dicke.

Alternativ kann das SMILE-Verfahren eingesetzt werden, bei der das Hornhautvolumen 21 durch einen kleinen Öffnungsschnitt entnommen wird, wie das in der DE 10 2007 019813 A1 beschrieben ist. In die so oder auf andere Weise erzeugte Tasche kann dann ein Implantat (Inlay) eingeführt werden.

Fig. 5 zeigt schematisch die Behandlungsvorrichtung 1, und anhand ihr soll die Bedeutung der Planungseinrichtung 16 näher erläutert werden. Die Behandlungsvorrichtung 1 weist in dieser Variante mindestens zwei Einrichtungen oder Module auf. Die bereits geschilderte Lasereinrichtung 4 gibt den Laserstrahl 6 auf das Auge 2 ab. Der Betrieb der Lasereinrichtung 4 erfolgt dabei, wie bereits geschildert, voll automatisch durch das Steuergerät 11, d.h. die Lasereinrichtung 4 startet auf ein entsprechendes Startsignal hin die Erzeugung und Ablenkung des Laserstrahls 6 und erzeugt dabei Schnittflächen, die auf die beschriebene Art und Weise aufgebaut sind. Die für den Betrieb erforderlichen Steuersignale empfängt die Lasereinrichtung 5 vom Steuergerät 11, dem zuvor entsprechende Steuerdaten bereitgestellt wurden. Dies erfolgt mittels der Planungseinrichtung 16, die in Fig. 5 lediglich exemplarisch als Bestandteil des Steuergeräts 11 gezeigt ist. Natürlich kann die Planungseinrichtung 16 auch eigenständig ausgebildet sein und drahtgebunden oder drahtlos mit der Steuereinrichtung 11 kommunizieren. Wesentlich ist dann lediglich, dass ein entsprechender Datenübertragungskanal zwischen der Planungseinrichtung 16 und dem Steuergerät 11 vorgesehen ist.

Die Planungseinrichtung 16 erzeugt einen Steuerdatensatz, der dem Steuergerät 11 zur Ausführung der augenchirurgischen Refraktionskorrektur zur Verfügung gestellt wird. Dabei verwendet die Planungseinrichtung Messdaten über die Hornhaut des Auges. Diese Daten stammen in der hier beschriebenen Ausführungsform aus einer Messeinrichtung 28, die das Auge 2 des Patienten 2 zuvor vermessen hat. Natürlich kann die Messeinrichtung 28 auf beliebige Art und Weise ausgebildet sein und die entsprechenden Daten an die Schnittstelle 29 der Planungseinrichtung 16 übermitteln.

Die Planungseinrichtung unterstützt nun den Bediener der Behandlungsvorrichtung 1 bei der Festlegung der Schnittfläche zur Isolierung des Hornhaut-Volumens 21 oder der Erzeugung einer Tasche (Pocket) für ein Implantat oder bei der Erstellung der erfindungsgemäßen Entlastungsschnitte. Dies kann bis zu einer vollautomatischen Festlegung der Schnitte gehen, die beispielsweise dadurch bewirkt werden kann, dass die Planungseinrichtung 16 aus den Messdaten beispielsweise das zu entnehmende Hornhaut-Volumen 21 ermittelt, dessen Begrenzungsflächen als Schnittflächen definiert und daraus entsprechende Steuerdaten für das Steuergerät 11 erzeugt. Am anderen Ende des Automatisierungsgrades kann die Planungseinrichtung 16 Eingabemöglichkeiten vorsehen, an denen ein Benutzer die Schnitte in Form von geometrischen oder optischen Parametern (Brechkräften bzw. Brechkraftänderungen) oder mechanischen Parametern (Elastizität) eingibt bzw. diese automatisiert aus Diagnosedaten abgeleitet werden. Zwischenstufen sehen Vorschläge für die Schnitte vor, welche die Planungseinrichtung 16 automatisch generiert und die von einem Bearbeiter dann modifizierbar sind. Grundsätzlich können all diejenigen Konzepte, die im vorstehend allgemeineren Beschreibungsteil bereits erläutert wurden, hier in der Planungseinrichtung 16 zur Anwendung kommen.

Um eine Behandlung durchzuführen, erzeugt die Planungseinrichtung 16 Steuerdaten für die Schnitterzeugung, die dann in der Behandlungsvorrichtung 1 verwendet werden.

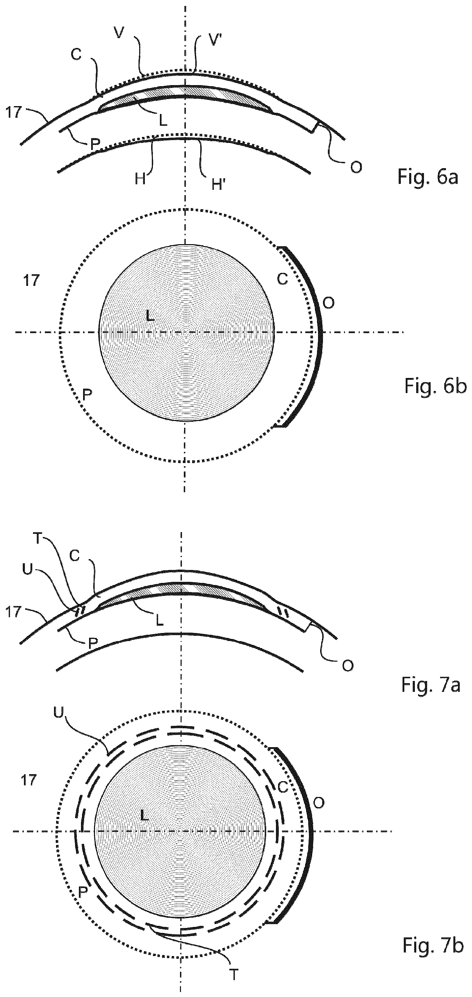

Fig. 6a zeigt eine Schemadarstellung einer Schnittgeometrie für ein Pocket nach dem Stand der Technik zur Verdeutlichung der geometrischen Verhältnisse im Querschnitt. Die Hornhaut 17 weist unter dem Cap C einen Pocketschnitt P mit einem Öffnungsschnitt O auf. Durch den Öffnungsschnitt O wurde ein Inlay L eingeführt. Das Inlay L verändert dadurch die Geometrie der Hornhaut 17 indem die Hornhautvorderseite V verformt wird. Eine mögliche Erklärung für die festgestellten Abweichungen zwischen Soll- und Ist-Refraktion nach Implantation eines Inlays könnte darin begründet sein, dass sich auch die Hornhautrückseite von H zu H' deformiert, damit aber die Hornhautvorderseite mechanisch reagiert und nur eine von der Zielverformung V abweichende Verformung V'aufweist, und so ein anderer als der gewünschte Refraktionszustand hergestellt wird. Fig. 6b zeigt eine Draufsicht auf die in Fig. 6a dargestellte Hornhaut.

Um dieser unerwünschten Abweichung von der Sollgeometrie entgegenzuwirken, werden zusätzliche Entlastungsschnitte in der Hornhaut über dem Cap C eingebracht.

Im Folgenden werden die erfindungsgemäßen Schnittgeometrien näher beschrieben.

Fig. 7a zeigt eine Schemadarstellung einer ersten erfindungsgemäßen Schnittgeometrie im Querschnitt. Dabei sind die Entlastungsschnitte T und U als Kreisbogensegmente ausgeführt welche symmetrisch um die optisch wirksame Zone des Auges verlaufen. Fig. 7b zeigt eine Draufsicht auf die in Fig. 7a dargestellte Hornhaut.

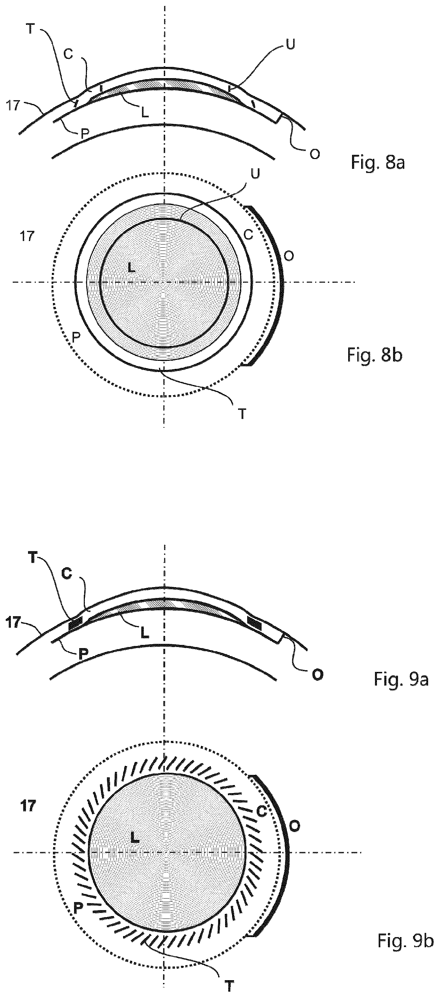

Fig. 8a zeigt eine Schemadarstellung einer zweiten erfindungsgemäßen Schnittgeometrie im Querschnitt. Dabei sind die Entlastungsschnitte T und U als vollständige Kreisbögen ausgeführt, welche außerhalb der optisch wirksamen Zone des Auges verlaufen. Die Entlastungsschnitte bewirken eine wirksamere Deformation der Hornhautvorderseite und vermindern die Deformation der Hornhautrückseite. Fig. 8b zeigt eine Draufsicht auf die in Fig. 8a dargestellte Hornhaut.

Fig. 9a zeigt eine Schemadarstellung einer weiteren erfindungsgemäßen Schnittgeometrie im Querschnitt. Dabei sind die Entlastungsschnitte T als schräg-radial verlaufende Schnitte ausgeführt. Diese Schnittvariante kann die im Cap beabsichtigte Gewebedehnung begünstigen. Fig. 9b zeigt eine Draufsicht auf die in Fig. 9a dargestellte Hornhaut.

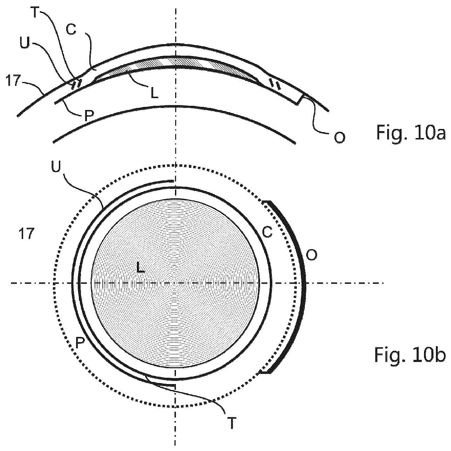

Fig. 10a zeigt eine Schemadarstellung einer weiteren Schnittgeometrie als Abwandlung der in Fig. 8 dargestellten Geometrie im Querschnitt. Hier wird durch einen nicht symmetrisch zur Augenachse verlaufenden Kreisbogenschnitt U ein Astigmatismus korrigiert. Fig. 10b zeigt eine Draufsicht auf die in Fig. 10a dargestellte Hornhaut

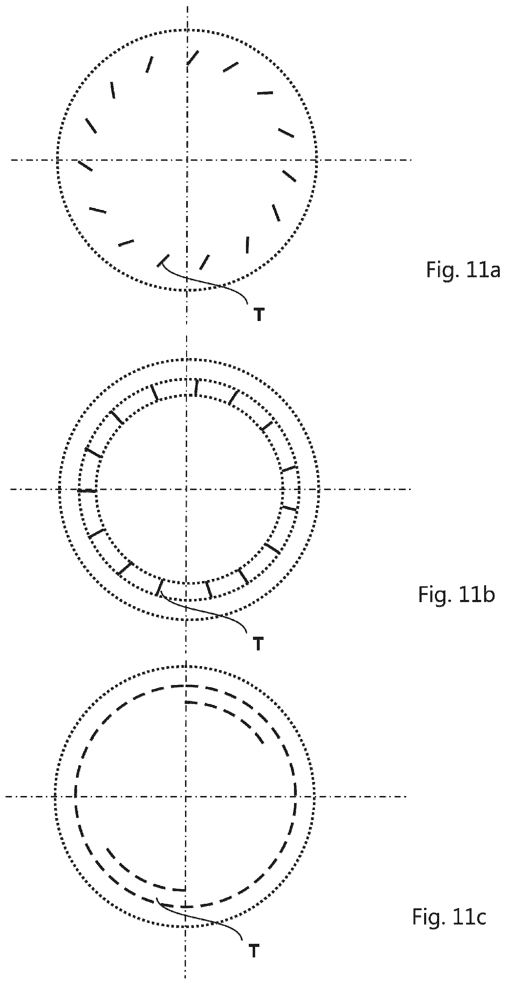

In Fig. 11 sind weitere mögliche Schnittgeometrien für die Entlastungsschnitte T dargestellt. Je nach Art und Ausmaß der angestrebten Gewebedehnung ermöglichen diese Schnittvarianten eine bessere Vorhersagbarkeit des Ergebnisses der Prozedur und eine beschleunigte Heilung.

In Fig. 11c ist die Astigmatismuskorrektur aus Fig. 10 in Verbindung mit der Schnittgestaltung aus Kreisbögen nach Fig. 8 dargestellt.

Wie den Figuren zu entnehmen ist, reichen die Entlastungschnitte T bzw. U nicht bis an die Hornhautvorderfläche, um das Risiko von Einreißen oder Aufklaffen zu vermeiden. Zur Absicherung der mechanischen Stabilität reichen sie auch nicht bis zum Inlay L. Bei gegebener Dicke d (zwischen 140µm und 200µm) des Caps C ist es meist günstig, wenn die Schnitte eine maximale Tiefenausdehnung von 0,8 x d haben. Allerdings kann es in besonderen Fällen auch vorteilhaft sein, wenn Entlastungsschnitte T bzw. U bis zum Inlay L reichen.

Zusätzlich sei noch angemerkt, dass die Behandlungsvorrichtung 1 bzw. die Planungseinrichtung 16 natürlich auch die Durchführung des zuvor allgemein erläuterten Verfahrens konkret realisiert.

Eine weitere Ausführungsform der Planungseinrichtung besteht in Form eines Computerprogramms bzw. eines entsprechenden Datenträgers mit einem Computerprogramm, der die Planungseinrichtung auf einem entsprechenden Computer realisiert, so dass die Eingabe der Messdaten über geeignete Datenübertragungsmittel an den Computer erfolgt und die Steuerdaten von diesem Computer an das Steuergerät 11 übertragen werden, wozu wiederum dem Fachmann bekannte Datenübertragungsmittel in Frage kommen.

## Patentansprüche

1. Planungseinrichtung (16) zum Erzeugen von Steuerdaten für eine Behandlungsvorrichtung (1) zur Augenchirurgie, die mittels einer Lasereinrichtung (4) zumindest einen Schnitt in der Hornhaut (17) erzeugt, wobei die Planungseinrichtung (16) Berechnungsmittel zum Festlegen mindestens eines Hornhaut-Entlastungsschnitts (T, U) aufweist, wobei die Berechnungsmittel den mindestens einen Hornhaut-Entlastungsschnitts basierend auf den Daten einer Refraktionskorrektur festlegen, und für die Hornhaut-Entlastungsschnitte (T, U) einen Steuerdatensatz zur Ansteuerung der Lasereinrichtung (4) erzeugen,
wobei die Berechnungsmittel den mindestens einen Hornhaut-Entlastungsschnitt (T, U) so bestimmen, dass nach Einbringen eines Implantats (L) in eine Tasche in der Hornhaut (17), mit einer Schnittgeometrie der Tasche aufweisend einen Pocketschnitt (P) unter einem Cap (C) der Dicke d und einen Öffnungsschnitt (O), bestehenden Refraktionsfehlern entgegengewirkt wird,
**dadurch gekennzeichnet, dass** der mindestens eine Hornhaut-Entlastungsschnitt (T, U) im Cap (C) verläuft, nicht an die Hornhautvorderfläche und nicht tiefer als bis zum Pocketschnitt (P) reicht und außerhalb der optisch wirksamen Zone verläuft.

2. Planungseinrichtung (16) nach Anspruch 1, wobei der mindestens eine Hornhaut-Entlastungsschnitt (T, U) eine maximale Tiefenausdehnung von 0,8 x d aufweist.

3. Planungseinrichtung (16) nach Anspruch 1 oder 2, wobei der mindestens eine Hornhaut-Entlastungsschnitt (T, U) als Kreisbogen, Kreisbogensegmente, radial verlaufende Schnitte oder schräg radial verlaufende Schnitte ausgeführt wird.

4. Behandlungsvorrichtung (1) zur Augenchirurgie, die
- eine Lasereinrichtung (4) aufweist, welche mittels Laserstrahlung gemäß Steuerdaten zumindest eine Schnittfläche in der Hornhaut (17) erzeugt, und
- eine Planungseinrichtung (16) zum Erzeugen der Steuerdaten aufweist, wobei die Planungseinrichtung (16) Berechnungsmittel zum Festlegen mindestens eines Hornhaut-Entlastungsschnitts (T, U) aufweist, wobei die Berechnungsmittel den mindestens einen Hornhaut-Entlastungsschnitt (T, U) basierend auf den Daten einer Refraktionskorrektur festlegen, und für den mindestens einen Hornhaut-Entlastungsschnitt (T, U) einen Steuerdatensatz zur Ansteuerung der Lasereinrichtung (4) erzeugen,
wobei die Planungseinrichtung (16) den mindestens einen Hornhaut-Entlastungsschnitt (T, U) so bestimmt, dass nach Einbringen eines Implantats (L) in eine Tasche in der Hornhaut (17), mit einer Schnittgeometrie der Tasche aufweisend einen Pocketschnitt (P) unter einem Cap (C) der Dicke d und einen Öffnungsschnitt (O), bestehenden Refraktionsfehlern entgegengewirkt wird,
**dadurch gekennzeichnet, dass** der mindestens eine Hornhaut-Entlastungsschnitt (T, U) im Cap (C) verläuft, nicht an die Hornhautvorderfläche und nicht tiefer als bis zum Pocketschnitt (P) reicht und außerhalb der optisch wirksamen Zone verläuft.

5. Behandlungseinrichtung (1) nach Anspruch 4, wobei der mindestens eine Hornhaut-Entlastungsschnitt (T, U) eine maximale Tiefenausdehnung von 0,8 x d aufweist.

6. Behandlungseinrichtung (1) nach Anspruch 4 oder 5, wobei der mindestens eine Hornhaut-Entlastungsschnitt (T, U) als Kreisbogen, Kreisbogensegmente, radial verlaufende Schnitte oder schräg radial verlaufende Schnitte ausgeführt wird.

7. Verfahren zum Erzeugen von Steuerdaten für eine Behandlungsvorrichtung (1) zur Augenchirurgie, die mittels einer Lasereinrichtung zumindest einen Schnitt in der Hornhaut (17) erzeugt, wobei das Verfahren durch folgende Schritte gekennzeichnet ist:
Bereitstellen von Hornhaut-Daten, basierend auf Daten einer Refraktionskorrektur, Festlegen mindestens eines Hornhaut-Entlastungsschnitts (T, U), und Erzeugen eines Steuerdatensatzes für den mindestens einen Hornhaut-Entlastungsschnitt (T, U) zur Ansteuerung der Lasereinrichtung (4),
wobei der mindestens eine Hornhaut-Entlastungsschnitt (T, U) so bestimmt wird, dass nach Einbringen eines Implantats (L) in eine Tasche in der Hornhaut (17), mit einer Schnittgeometrie der Tasche aufweisend einen Pocketschnitt (P) unter einem Cap (C) der Dicke d und einen Öffnungsschnitt (O), bestehenden Refraktionsfehlern entgegengewirkt wird,
**dadurch gekennzeichnet, dass** der mindestens eine Hornhaut-Entlastungsschnitt (T, U) im Cap (C) verläuft, nicht an die Hornhautvorderfläche und nicht tiefer als bis zum Pocketschnitt (P) reicht und außerhalb der optisch wirksamen Zone verläuft.

8. Verfahren nach Anspruch 7, wobei der mindestens eine Hornhaut-Entlastungsschnitt (T, U) eine maximale Tiefenausdehnung von 0,8 x d aufweist.

9. Verfahren nach Anspruch 7 oder 8, wobei der mindestens eine Hornhaut-Entlastungsschnitt (T, U) als Kreisbogen, Kreisbogensegmente, radial verlaufende Schnitte oder schräg radial verlaufende Schnitte ausgeführt wird.

10. Computerprogrammprodukt mit Programm-Code der bei Ausführung auf einem Computer das Verfahren nach einem der Ansprüche 7 bis 9 ausführt.

11. Datenträger mit einem Computerprogrammprodukt nach Anspruch 10.

## Claims

1. Planning device (16) for generating control data for a treatment apparatus (1) for eye surgery which generates at least one incision in the cornea (17) by means of a laser device (4), wherein the planning device (16) comprises calculation means for defining at least one corneal relief incision (T, U), wherein the calculation means define the at least one corneal relief incision based on the data of a refractive correction, and generate a control data record for controlling the laser device (4) for the corneal relief incisions (T, U),
wherein the calculation means determine the at least one corneal relief incision (T, U) in such a way that refractive errors present following the insertion of an implant (L) into a pocket in the cornea (17), with an incision geometry of the pocket comprising a pocket incision (P) under a cap (C) of thickness d and an opening incision (O), are countervailed,
**characterized in that** the at least one corneal relief incision (T, U) extends in the cap (C), does not reach the anterior corneal surface and does not reach deeper than the pocket incision (P) and extends outside the optically effective zone.

2. Planning device (16) according to Claim 1, wherein the at least one corneal relief incision (T, U) has a maximum extension in depth of 0.8 x d.

3. Planning device (16) according to Claim 1 or 2, wherein the at least one corneal relief incision (T, U) is embodied as a circular arc, as circular arc segments, as radially extending incisions or as obliquely-radially extending incisions.

4. Treatment apparatus (1) for eye surgery, which
- comprises a laser device (4) which according to control data generates at least one cut surface in the cornea (17) by means of laser radiation, and
- comprises a planning device (16) for generating the control data, wherein the planning device (16) comprises calculation means for defining at least one corneal relief incision (T, U), wherein the calculation means define the at least one corneal relief incision (T, U) based on the data of a refractive correction, and generate a control data record for controlling the laser device (4) for the at least one corneal relief incision (T, U),
wherein the planning device (16) determines the at least one corneal relief incision (T, U) in such a way that refractive errors present following the insertion of an implant (L) into a pocket in the cornea (17), with an incision geometry of the pocket comprising a pocket incision (P) under a cap (C) of thickness d and an opening incision (O), are countervailed,
**characterized in that** the at least one corneal relief incision (T, U) extends in the cap (C), does not reach the anterior corneal surface and does not reach deeper than the pocket incision (P) and extends outside the optically effective zone.

5. Treatment device (1) according to Claim 4, wherein the at least one corneal relief incision (T, U) has a maximum extension in depth of 0.8 x d.

6. Treatment device (1) according to Claim 4 or 5, wherein the at least one corneal relief incision (T, U) is embodied as a circular arc, as circular arc segments, as radially extending incisions or as obliquely-radially extending incisions.

7. Method for generating control data for a treatment apparatus (1) for eye surgery which generates at least one incision in the cornea (17) by means of a laser device, wherein the method is **characterized by** the following steps:
providing corneal data based on data of a refractive correction, defining at least one corneal relief incision (T, U) and generating a control data record for the at least one corneal relief incision (T, U) for the purpose of controlling the laser device (4),
wherein the at least one corneal relief incision (T, U) is determined in such a way that refractive errors present following the insertion of an implant (L) into a pocket in the cornea (17), with an incision geometry of the pocket comprising a pocket incision (P) under a cap (C) of thickness d and an opening incision (O), are countervailed,
**characterized in that** the at least one corneal relief incision (T, U) extends in the cap (C), does not reach the anterior corneal surface and does not reach deeper than the pocket incision (P) and extends outside the optically effective zone.

8. Method according to Claim 7, wherein the at least one corneal relief incision (T, U) has a maximum extension in depth of 0.8 x d.

9. Method according to Claim 7 or 8, wherein the at least one corneal relief incision (T, U) is embodied as a circular arc, as circular arc segments, as radially extending incisions or as obliquely-radially extending incisions.

10. Computer program product having program code which upon execution on a computer carries out the method according to any of Claims 7 to 9.

11. Data medium having a computer program product according to Claim 10.

## Revendications

1. Dispositif de planification (16) destiné à générer des données de commande pour un appareil de traitement (1) pour la chirurgie ophthalmique, qui réalise au moins une incision dans la cornée (17) à l'aide d'un dispositif laser (4), le dispositif de planification (16) comportant des moyens de calcul destinés à déterminer au moins une incision de relâchement de cornée (T, U), les moyens de calcul déterminant l'au moins une incision de relâchement de cornée sur la base des données d'une correction de réfraction, et générant pour les incisions de relâchement de cornée (T, U) un ensemble de données de commande destiné à commander le dispositif laser (4),
les moyens de calcul déterminant l'au moins une incision de relâchement de cornée (T, U) de telle sorte que des erreurs de réfraction existantes soient neutralisées après l'insertion d'un implant (L) dans une poche dans la cornée (17), avec une géométrie d'incision de la poche présentant une incision de poche (P) sous un capuchon (C) d'épaisseur d et une incision d'ouverture (O),
**caractérisé en ce que** l'au moins une incision de relâchement de cornée (T, U) s'étend dans le capuchon (C), n'atteint pas la surface antérieure de la cornée et n'est pas plus profonde que l'incision de poche (P) et s'étend en dehors de la zone optiquement efficace.

2. Appareil de planification (16) selon la revendication 1, l'au moins une incision de relâchement de cornée (T, U) présentant une extension maximale en profondeur de 0,8 x d.

3. Appareil de planification (16) selon la revendication 1 ou 2, l'au moins une incision de relâchement de cornée (T, U) étant réalisée sous la forme d'un arc de cercle, de segments d'arc de cercle, de coupes radiales ou de coupes obliques radiales.

4. Appareil de traitement (1) destiné à la chirurgie ophthalmique, lequel
- comporte un dispositif laser (4) qui génère au moins une surface d'incision dans la cornée (17) à l'aide d'un rayonnement laser en fonction de données de commande, et
- comporte un dispositif de planification (16) destiné à générer les données de commande, le dispositif de planification (16) comportant des moyens de calcul destinés à déterminer au moins une incision de relâchement de cornée (T, U), les moyens de calcul déterminant l'au moins une incision de relâchement de cornée (T, U) sur la base des données d'une correction de réfraction, et générant un ensemble de données de commande destiné à commander le dispositif laser (4) pour l'au moins une incision de décharge de cornée (T, U),
le dispositif de planification (16) déterminant l'au moins une incision de relâchement de cornée (T, U) de telle sorte que des erreurs de réfraction existantes soient neutralisées après l'insertion d'un implant (L) dans une poche dans la cornée (17), avec une géométrie d'incision de la poche présentant une incision de poche (P) sous un capuchon (C) d'épaisseur d et une incision d'ouverture (O),
**caractérisé en ce que** l'au moins une incision de relâchement de cornée (T, U) s'étend dans le capuchon (C), n'atteint pas la surface antérieure de la cornée et n'est pas plus profonde que l'incision de poche (P) et s'étend en dehors de la zone optiquement efficace.

5. Appareil de traitement (1) selon la revendication 4, l'au moins une incision de relâchement de cornée (T, U) présentant une extension maximale en profondeur de 0,8 x d.

6. Appareil de traitement (1) selon la revendication 4 ou 5, l'au moins une incision de relâchement de cornée (T, U) étant réalisée sous la forme d'un arc de cercle, de segments d'arc de cercle, de coupes radiales ou de coupes obliques radiales.

7. Procédé de génération de données de commande destinées à un appareil de traitement (1) pour la chirurgie ophthalmique, qui réalise au moins une incision dans la cornée (17) à l'aide d'un dispositif laser, le procédé étant **caractérisé par** les étapes suivantes :
fournir des données de cornée sur la base de données de correction de réfraction, définir au moins une incision de décharge de cornée (T, U), et générer un ensemble de données de commande pour ladite au moins une incision de relâchement de cornée (T, U) afin de commander le dispositif laser (4),
l'au moins une incision de relâchement de cornée (T, U) étant déterminée de telle sorte que des erreurs de réfraction existantes soient neutralisées après l'insertion d'un implant (L) dans une poche dans la cornée (17), avec une géométrie d'incision de la poche présentant une incision de poche (P) sous un capuchon (C) d'épaisseur d et une incision d'ouverture (O),
**caractérisé en ce que** l'au moins une incision de relâchement de cornée (T, U) s'étend dans le capuchon (C), n'atteint pas la surface antérieure de la cornée et n'est pas plus profonde que l'incision de poche (P) et s'étend en dehors de la zone optiquement efficace.

8. Procédé selon la revendication 7, l'au moins une incision de relâchement de cornée (T, U) présentant une extension maximale en profondeur de 0,8 x d.

9. Procédé selon la revendication 7 ou 8, l'au moins une incision de relâchement de cornée (T, U) étant réalisée sous la forme d'un arc de cercle, de segments d'arc de cercle, de coupes radiales ou de coupes obliques radiales.

10. Produit programme informatique comprenant un code de programme qui, lorsqu'il est exécuté sur un ordinateur, met en œuvre le procédé selon l'une des revendications 7 à 9.

11. Support de données comprenant un produit programme informatique selon la revendication 10.
